# EUROPEAN PATENT APPLICATION

(11) **EP 4 180 502 A1**
(43) Date of publication of application: **17.05.2023**
(21) Application number: 21838292.7
(22) Date of filing: 08.07.2021
(51) Int. Cl.: C09K 17/32, A01G 22/20, C05G 3/80

(54) **SOIL CONDITIONER AND USE THEREOF**

(30) Priority: 08.07.2020 JP 2020117949
(71) Applicant: Japan International Research Center for Agricultural Sciences, Tsukuba-shi Ibaraki 305-8686 (JP)
(72) Inventor: YOSHIHASHI Tadashi, Tsukuba-shi, Ibaraki 305-8686 (JP); SUBBARAO Guntur Venkata, Tsukuba-shi, Ibaraki 305-8686 (JP); NAKAHARA Kazuhiko, Tsukuba-shi, Ibaraki 305-8686 (JP); ONO Hiroshi, Tsukuba-shi, Ibaraki 305-8517 (JP)
(74) Representative: Cabinet Becker et Associés
(86) International application number: PCT/JP2021/025793
(87) International publication number: WO 2022/009955

(57) **Abstract**

Provided is a soil conditioner that comprises, as an active ingredient, a compound represented by general formula (1), a salt thereof or a solvate of the same. In general formula (1), R¹ to R⁶ independently represent an optionally substituted hydrocarbon group having 1-5 carbon atoms, an optionally substituted alkoxy group having 1-5 carbon atoms, an amino group, a hydroxy group, a halogen atom or a hydrogen atom.

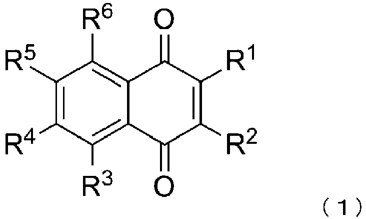

## Description

### [Technical Field]

The present invention relates to a soil conditioner and use thereof. More specifically, the present invention relates to a soil conditioner, a fertilizer, a method for producing an active ingredient of the soil conditioner, and a nitrification inhibition method. Priority is claimed on Japanese Patent Application No. 2020-117949, filed July 8, 2020, the content of which is incorporated herein by reference.

### [Background Art]

Ammonia-state nitrogen contained in a chemical fertilizer, such as ammonia, an ammonium salt, and urea, and ammonia-state nitrogen generated by decomposition of an organic fertilizer are easily converted into nitrate-state nitrogen in the soil, particularly under oxidative conditions in the surface layer or the like of fields and paddy fields.

This action, called nitrification, is caused by the action of nitrifying bacteria such as a nitrite bacterium and a nitrate bacterium, and archaea. The generated nitrous acid and nitrate ions are not adsorbed by soil colloids but run off and are released into the underground water as nitrate nitrogen or into the atmosphere as nitrous oxide, which has become a problem as a powerful greenhouse gas, due to the denitrifying action in the soil. As a result, the utilization rate of fertilized nitrogen fertilizers by crops is very low in oxidative soil conditions with a strong nitrification action, and the diffusion of nitrate nitrogen and nitrous oxide generated by nitrification into the environment is also a cause of pollution of the natural environment.

However, since fertilization using chemical fertilizers in which nitrogen in the air is chemically fixed is the most reliable method of dramatically increasing crop production, considerable amounts of nitrogen fertilizers have been put into farmland. Therefore, although the environmental conditions are limited, compounds having a nitrification inhibitory activity, such as nitrapyrin (2-chloro-6-trichloromethylpyridine) and a synthetic agent, for example, dicyandiamide described in Patent Document 1, or fertilizers containing these have been used in the related art.

In tropical regions, it is known that nitrification is inhibited in soil in which creeping signal grass (Brachiaria humidicola), which is a tropical grass, grows (Non-Patent Document 1), and the invention exemplified in Patent Document 2, which utilizes this phenomenon, is known. Further, in general grains, it is known that sorgoleone secreted from the roots of sorghum has a nitrification inhibitory activity (Non-Patent Document 2). Further, the invention exemplified in Patent Document 3, which utilizes a nitrification inhibitory activity of fatty acids and derivatives of fatty acids, is also known.

### [Citation List]

### [Non-Patent Documents]

[Non-Patent Document 1]
   G.V. Subbarao, T. Ishikawa, O. Ito, K. Nakahara, H.Y. Wang, W.L. Berry, A bioluminescence assay to detect nitrification inhibitors released from plant roots: a case study with Brachiaria humidicola. Plant and Soil 288(1-2), 101-112, 2006.
[Non-Patent Document 2]
   T. Tesfamariam, H. Yoshinaga, S.P. Deshpande, P.S. Rao, K.L. Sahrawat, Y. Ando, K, Nakahara, C.T. Hash, G.V. Subbarao, Biological nitrification inhibition in sorghum; the role of sorgoleone production. Plant and Soil 379 (1-2), 325-335, 2014.

### [Patent Documents]

[Patent Document 1]
   Japanese Unexamined Patent Application, First Publication No. H11-278973
[Patent Document 2]
   Japanese Patent No. 5408478
[Patent Document 3]
   Japanese Patent No. 5067520

### [Summary of Invention]

### [Technical Problem]

However, it is known that nitrapyrin is highly volatile and has almost no effect under conditions in which the ground temperature is 20°C or higher, and thus dicyandiamide has been mainly used.

Dicyandiamide can also be used at a high temperature as compared with nitrapyrin. However, since dicyandiamide should be used at a high concentration and is expensive, agricultural production costs are greatly affected. As a result, the areas where dicyandiamide can be used are limited.

In addition, as a result of dicyandiamide being detected in dairy products produced in New Zealand in 2013, New Zealand has come to regulate the use of dicyandiamide-containing fertilizers in pastures. Since nitrification inhibitory substances in the related art are synthetic agents and have not been found in nature, there are increasing expectations for an alternative nitrification inhibitory substance from natural products, which can be assumed to have a relatively low environmental load.

There has been no report on the nitrification inhibition in maize, which is an important grain that is produced in plowed fields where the soil is in an oxidized state. In addition, there has been no report on the nitrification inhibitory activity of 1,4-naphthoquinones, which are isolated from various plants and have various physiological activities.

In consideration of the above problems, an object of the present invention is to provide a soil conditioner, a nitrification inhibitor, a fertilizer, and a nitrification inhibition method which can be used over wide areas from tropical zones to temperate zones and in which a compound easily obtained from a material of natural origin or a derivative thereof is used. In addition, another object is to carry out inhibition of soil nitrification by utilizing an appropriate maize strain since it was found that the content of the above substance differs greatly between maize strains.

### [Solution to Problem]

The present invention includes the following aspects.
[1] A soil conditioner containing, as an active ingredient, a compound represented by Formula (1) or a solvate of the compound or the salt thereof. 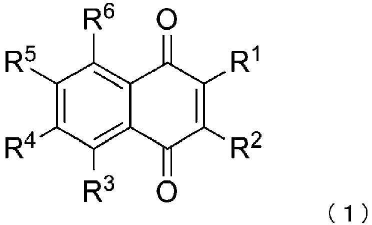 [In Formula (1), R¹ to R⁶ each independently represents a hydrocarbon group having 1 to 5 carbon atoms, which may have a substituent, an alkoxy group having 1 to 5 carbon atoms, which may have a substituent, an amino group, a hydroxy group, a halogen atom, or a hydrogen atom.]
[2] The soil conditioner according to [1], in which the compound represented by Formula (1) is one or more selected from the group consisting of 1,4-naphthoquinone, 2-methyl-1,4-naphthoquinone, 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2-methoxy-1,4-naphthoquinone, 2-amino-3-chloro-1,4-naphthoquinone, 2,3-dichloro-5,8-dihydroxy-1,4-naphthoquinone, 6-methyl-1,4-naphthoquinone, 2-chloro-1,4-naphthoquinone, and 2,7-dimethoxy-1,4-naphthoquinone.
[3] The soil conditioner according to [1] of [2], in which the soil conditioner has an effect of inhibiting nitrification.
[4] A fertilizer containing the soil conditioner according to any one of [1] to [3].
[5] A production method for an active ingredient of the soil conditioner according to any one of [1] to [3], the production method including:
   soaking a root surface part of a maize in an organic solvent to obtain a root exudate; and
   purifying the root exudate by chromatography.[6] A nitrification inhibition method for soil, including cultivating a plant that generates an active ingredient of the soil conditioner according to any one of [1] to [3].

### [Advantageous Effects of Invention]

According to the present invention, it is possible to provide a soil conditioner, a nitrification inhibitor, a fertilizer, and a nitrification inhibition method which uses a compound capable of being used over wide areas from tropical zones to temperate zones and is easily obtained from a material of natural origin, or a derivative of the compound.

### [Brief Description of Drawings]

FIG. 1 is an ultraviolet visible absorption spectrum of a generated compound from an extraction liquid of the surface of a maize root.
FIG. 2 is an electrospray ionization mass spectrum of a generated compound from an extraction liquid of the surface of a maize root.
FIG. 3 is a ¹H-NMR spectrum of a generated compound from an extraction liquid on the surface of a maize root.
FIG. 4 is a ¹³C-NMR spectrum of a generated compound from an extraction liquid on the surface of a maize root.
FIG. 5 is a dose response curve of nitrification inhibition by 2,7-dimethoxy-1,4-naphthoquinone.
FIG. 6 is a graph showing the content of 2,7-dimethoxy-1,4-naphthoquinone obtained from the roots of 11 strains of maize.

### [Description of Embodiments]

Due to the observation that a hydrophobic extract of a root surface part of maize has a strong nitrification inhibitory action, the inventors of the present invention anticipated that some kind of nitrification inhibitory substance would be released from the maize roots and carried out diligent research.

As a result, they isolated and obtained a nitrification inhibitory substance, identified the chemical structure thereof, and furthermore, confirmed that the isolated compound has an effect of inhibiting nitrification, whereby the invention was completed. That is, the isolated compound according to the present invention is a 1,4-naphthoquinone derivative represented by Formula (2), that is, 2,7-dimethoxy-1,4-naphthoquinone (hereinafter, may be referred to as "zeanone").

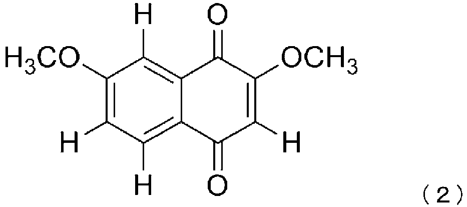

In addition, as will be described later in Examples, the nitrification inhibitory activity of derivatives having a structure similar to 2,7-dimethoxy-1,4-naphthoquinone (zeanone) was investigated. As a result, the derivatives shown in Table 1 were found to have nitrification inhibitory activity. These 1,4-naphthoquinone derivatives can also be used in nitrification inhibition in the same manner as zeanone.

In Table 1, R¹ to R⁶ are each R¹ to R⁶ in General Formula (1), respectively.

**[Table 1]**

| Substance name | R¹ | R² | R³ | R⁴ | R⁵ | R⁶ |
|---|---|---|---|---|---|---|
| 1,4-Naphthoquinone | H | H | H | H | H | H |
| 2-Methyl-1,4-naphthoquinone | CH₃ | H | H | H | H | H |
| 5-Hydroxy-1,4-naphthoquinone | H | H | OH | H | H | H |
| 5,8-Dihydroxy-1,4-naphthoquinone | H | H | OH | H | H | OH |
| 5-Hydroxy-2-methyl-1,4-naphthoquinone | CH₃ | H | OH | H | H | H |
| 2,3-Dichloro-1,4-naphthoquinone | Cl | Cl | H | H | H | H |
| 2-Methoxy-1,4-naphthoquinone | CH₃O | H | H | H | H | H |
| 2-Amino-3-chloro-1,4-naphthoquinone | NH₂ | Cl | H | H | H | H |
| 2,3-Dichloro-5,8-dihydroxy-1,4-naphthoquinone | Cl | Cl | OH | H | H | OH |
| 6-Methyl-1,4-naphthoquinone | H | H | H | CH₃ | H | H |
| 2-Chloro-1,4-naphthoquinone | Cl | H | H | H | H | H |
| Zeanone | CH₃O | H | H | H | CH₃O | H |

2,7-Dimethoxy-1,4-naphthoquinone (zeanone) is a substance isolated from maize which is widely cultivated under fertilization conditions and produced in a plowed field of which the soil is in an oxidized state and is prone to nitrification. Due to having low volatility, zeanone is conceived to be not easily affected by the environmental temperature in terms of nitrification inhibition, and it has high general-purpose properties as compared with the already used nitrapyrin. A large number of the derivatives of this compound have also been reported as plant components, and it was confirmed that they have a nitrification inhibitory action, as will be described later in Examples. In a case of selecting an appropriate plant containing these substances, it is possible to inhibit soil nitrification and enhance the nitrogen utilization efficiency, whereby it is possible to prevent the adverse effects due to nitrification, such as the generation of nitrous oxide from the soil and the outflow of nitrate nitrogen into underground water.

### [Soil conditioner]

In one embodiment, the present invention provides a soil conditioner containing, as an active ingredient, a compound represented by Formula (1) or a solvate of the compound or the salt thereof.

By containing this active ingredient, the soil conditioner according to the present embodiment can obtain a practically useful effect of inhibiting nitrification.

In the present specification, the compound represented by Formula (1) may be referred to as a 1,4-naphthoquinone derivative.

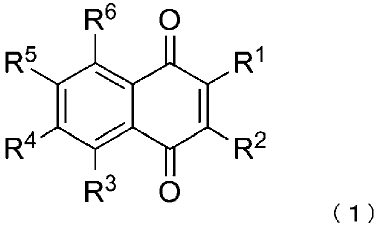

[In Formula (1), R¹ to R⁶ each independently represents a hydrocarbon group having 1 to 5 carbon atoms, which may have a substituent, an alkoxy group having 1 to 5 carbon atoms, which may have a substituent, an amino group, a hydroxy group, a halogen atom, or a hydrogen atom.]

In R¹ to R⁶ in General Formula (1), the hydrocarbon group having 1 to 5 carbon atoms, which may have a substituent, is a saturated hydrocarbon group or an unsaturated hydrocarbon group, and is preferably a saturated hydrocarbon group.

The hydrocarbon group having 1 to 5 carbon atoms, which may have a substituent, may be linear, branched, or cyclic. The hydrocarbon group is preferably linear or branched, and is more preferably linear.

The hydrocarbon group having 1 to 5 carbon atoms is preferably an alkyl group having 1 to 5 carbon atoms.

Examples of the alkyl group having 1 to 5 carbon atoms include a methyl group, an ethyl group, a propyl group, an isopropyl group, an n-butyl group, an isobutyl group, a tert-butyl group, a pentyl group, an isopentyl group, and a neopentyl group.

The hydrocarbon group preferably has 1 to 3 carbon atoms.

The hydrocarbon group is more preferably a methyl group or an ethyl group and still more preferably a methyl group.

In a case where the hydrocarbon group has a substituent, the substituent includes a halogen atom.

In R¹ to R⁶ in General Formula (1), examples of the alkoxy group (-OR) having 1 to 5 carbon atoms, which may have a substituent, include those in which the R portion of -OR is the same as the above-described hydrocarbon group having 1 to 5 carbon atoms, which may have a substituent.

The alkoxy group (-OR) is more preferably a methoxy group or an ethoxy group and still more preferably a methoxy group.

In R¹ to R⁶ in General Formula (1), the halogen atom may be a fluorine atom, a chlorine atom, a bromine atom, or an iodine atom, and is preferably a chlorine atom.

In General Formula (1), R¹ is preferably a group other than the hydroxy group.

As the compound represented by General Formula (1), one kind may be used alone, or two or more kinds may be used.

Suitable examples of the compound represented by General Formula (1) include the following compound (1-1), compound (1-2), and compound (1-3), from the viewpoint of nitrification inhibitory activity.
Compound (1 -1): A compound in which at least one of R³ to R⁶ is a hydroxy group.
Compound (1-2): A compound in which R¹ or R² is a halogen atom.
Compound (1-3): A compound in which at least one of R³ to R⁶ is an alkyl group having 1 to 5 carbon atoms, which may have a substituent.

These will be described in detail.

### <Compound (1-1)>

The compound (1-1) is a compound represented by General Formula (1) in which at least one of R³ to R⁶ is a hydroxy group.

In the compound (1-1), R³ is preferably a hydroxy group.

In a case where R³ is a hydroxy group, each of R¹ and R² may be a hydrocarbon group having 1 to 5 carbon atoms, which may have a substituent, or may be a hydrogen atom.

In a case where both R¹ and R² are a hydrogen atom, it is preferable that R⁶ be a hydroxy group or a hydrogen atom.

Further, it is preferable that each of R⁴ and R⁵ be a hydrogen atom, and it is more preferable that both R⁴ and R⁵ be a hydrogen atom.

Alternatively, in a case where R³ is a hydroxy group, the compound (1-1) may be such a compound as follows.

It is preferable that each of R¹ and R² be a hydrocarbon group having 1 to 5 carbon atoms, which may have a substituent, it is more preferable that any one of R¹ and R² be a hydrocarbon group having 1 to 5 carbon atoms, which may have a substituent, and the other thereof be a hydrogen atom, and it is still more preferable that R¹ be a hydrocarbon group having 1 to 5 carbon atoms, which may have a substituent, and R² be a hydrogen atom. Further, R⁶ is preferably a hydrogen atom.

In R¹ and R², the hydrocarbon group having 1 to 5 carbon atoms, which may have a substituent, is a saturated hydrocarbon group or an unsaturated hydrocarbon group, and is preferably a saturated hydrocarbon group.

The hydrocarbon group having 1 to 5 carbon atoms, which may have a substituent, may be linear, branched, or cyclic. The hydrocarbon group is preferably linear or branched, and is more preferably linear.

The hydrocarbon group having 1 to 5 carbon atoms is preferably an alkyl group having 1 to 5 carbon atoms.

Examples of the alkyl group having 1 to 5 carbon atoms include a methyl group, an ethyl group, a propyl group, an isopropyl group, an n-butyl group, an isobutyl group, a tert-butyl group, a pentyl group, an isopentyl group, and a neopentyl group.

The hydrocarbon group preferably has 1 to 3 carbon atoms.

The hydrocarbon group is more preferably a methyl group or an ethyl group and still more preferably a methyl group.

In a case where the hydrocarbon group has a substituent, the substituent includes a halogen atom.

Further, it is preferable that each of R⁴ and R⁵ be a hydrogen atom, and it is more preferable that both R⁴ and R⁵ be a hydrogen atom.

Alternatively, in a case where R³ is a hydroxy group, the compound (1-1) may be such a compound as follows.

Each of R¹ to R² may be a halogen atom or a hydrogen atom.

Each of R¹ and R² is preferably a halogen atom, and both R¹ and R² are preferably a halogen atom.

It is preferable that both R¹ and R² be a halogen atom and furthermore, R⁶ be a hydroxy group.

The halogen atom may be a fluorine atom, a chlorine atom, a bromine atom, or an iodine atom, and is preferably a chlorine atom.

Further, it is preferable that each of R⁴ and R⁵ be a hydrogen atom, and it is more preferable that both R⁴ and R⁵ be a hydrogen atom.

Examples of the compound (1-1) include 5-hydroxy-1,4-naphthoquinone, 5,8-dihydroxy-1,4-naphthoquinone, 5-hydroxy-2-methyl-1,4-naphthoquinone, and 2,3-dichloro-5,8-dihydroxy-1,4-naphthoquinone.

Among these, 5-hydroxy-2-methyl-1,4-naphthoquinone and 2,3-dichloro-5,8-dihydroxy-1,4-naphthoquinone are preferable.

### <Compound (1-2)>

The compound (1-2) is a compound represented by General Formula (1) in which R¹ or R² is a halogen atom.

In R¹ to R², it is preferable that at least one of R¹ and R² be a halogen atom, and it is more preferable that both R¹ and R² be a halogen atom.

The halogen atom may be a fluorine atom, a chlorine atom, a bromine atom, or an iodine atom, and is preferably a chlorine atom.

Further, R³ is preferably a hydroxy group or a hydrogen atom and more preferably a hydroxy group.

Further, it is preferable that each of R⁴ and R⁵ be a hydrogen atom, and it is more preferable that both R⁴ and R⁵ be a hydrogen atom.

Further, R⁶ is preferably a hydroxy group or a hydrogen atom and more preferably a hydroxy group.

Examples of the compound (1-2) include 2,3-dichloro-1,4-naphthoquinone, 2-amino-3-chloro-1,4-naphthoquinone, 2,3-dichloro-5,8-dihydroxy-1 ,4-naphthoquinone, and 2-chloro-1,4-naphthoquinone.

Among these, 2,3-dichloro-1,4-naphthoquinone, 2,3-dichloro-5,8-dihydroxy-1,4-naphthoquinone, and 2-chloro-1,4-naphthoquinone are preferable.

Among these, 2,3-dichloro-5,8-dihydroxy-1,4-naphthoquinone is more preferable.

### <Compound (1-3)>

The compound (1-3) is a compound represented by General Formula (1) in which at least one of R³ to R⁶ is a hydrocarbon group having 1 to 5 carbon atoms, which may have a substituent.

In the compound (1-3), each of R⁴ and R⁵ is preferably a hydrocarbon group having 1 to 5 carbon atoms, which may have a substituent, and it is more preferable that any one of R⁴ and R⁵ be a hydrocarbon group having 1 to 5 carbon atoms, which may have a substituent, and the other thereof be a hydrogen atom.

The hydrocarbon group having 1 to 5 carbon atoms, which may have a substituent, is a saturated hydrocarbon group or an unsaturated hydrocarbon group, and is preferably a saturated hydrocarbon group.

The hydrocarbon group having 1 to 5 carbon atoms, which may have a substituent, may be linear, branched, or cyclic. The hydrocarbon group is preferably linear or branched, and is more preferably linear.

The hydrocarbon group having 1 to 5 carbon atoms is preferably an alkyl group having 1 to 5 carbon atoms.

Examples of the alkyl group having 1 to 5 carbon atoms include a methyl group, an ethyl group, a propyl group, an isopropyl group, an n-butyl group, an isobutyl group, a tert-butyl group, a pentyl group, an isopentyl group, and a neopentyl group.

The hydrocarbon group preferably has 1 to 3 carbon atoms.

The hydrocarbon group is more preferably a methyl group or an ethyl group and still more preferably a methyl group.

In a case where the hydrocarbon group has a substituent, the substituent includes a halogen atom.

Further, it is preferable that each of R³ and R⁶ be a hydrogen atom, and it is more preferable that both R³ and R⁶ be a hydrogen atom.

Further, it is preferable that each of R¹ and R² be a hydrogen atom, and it is more preferable that both R¹ and R² be a hydrogen atom.

Examples of the compound (1-3) include 6-methyl-1,4-naphthoquinone.

Among the above-described compound (1-1), compound (1-2), and compound (1-3), the compound represented by General Formula (1) is preferably the compound (1-1) or the compound (1-2) and more preferably the compound (1-1) from the viewpoint of further enhancing the nitrification inhibitory activity of the compound.

The compound represented by General Formula (1) may be one or more selected from the group consisting of 1,4-naphthoquinone, 2-methyl-1,4-naphthoquinone, 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2-methoxy-1,4-naphthoquinone, 2-amino-3-chloro-1,4-naphthoquinone, 2,3-dichloro-5,8-dihydroxy-1,4-naphthoquinone, 6-methyl-1,4-naphthoquinone, 2-chloro-1,4-naphthoquinone, and 2,7-dimethoxy-1,4-naphthoquinone (zeanone), which are shown in Table 1.

As will be described later in Examples, the inventors of the present invention measured the nitrification inhibitory activity of the above-described compounds.

From the viewpoint that a compound having a high nitrification inhibitory activity is preferable, the compound represented by General Formula (1) is more preferably one or more selected from the group consisting of 1,4-naphthoquinone, 2-methyl-1,4-naphthoquinone, 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2,3-dichloro-5,8-dihydroxy-1,4-naphthoquinone, 6-methyl-1,4-naphthoquinone, and 2-chloro-1,4-naphthoquinone, more preferably one or more selected from the group consisting of 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2,3-dichloro-5,8-dihydroxy-1,4-naphthoquinone, and 6-methyl-1,4-naphthoquinone, and still more preferably one or more selected from the group consisting of 5-hydroxy-2-methyl-1,4-naphthoquinone and 2,3-dichloro-5,8-dihydroxy-1,4-naphthoquinone.

In the cultivation of agricultural crops, it is important to inhibit soil nitrification and enhance the nitrogen utilization efficiency. It is preferable that the nitrification inhibitor be of natural origin and be easy to use.

As described above, since 2,7-dimethoxy-1,4-naphthoquinone (zeanone) is secreted from maize roots, it is possible to inhibit soil nitrification by cultivating maize. In addition, zeanone has low volatility and is not easily affected by the environmental temperature.

From this point of view, the compound represented by General Formula (1) is preferably 2,7-dimethoxy-1,4-naphthoquinone (zeanone).

The salt is not particularly limited as long as it is agriculturally acceptable. Examples of such a salt include inorganic acid salts such as a hydrochloride, a sulfate, and a nitrate; organic acid salts such as an acetate and a methanesulfonate; alkali metal salts such as a sodium salt and a potassium salt; alkaline earth metal salts such as a magnesium salt and a calcium salt; and quaternary ammonium salts such as dimethyl ammonium and triethyl ammonium.

Zeanone represented by Formula (2) is a newly confirmed compound, which is of natural origin. As will be described later in Examples, the inventors of the present invention have found that zeanone has an excellent nitrification inhibitory action. Zeanone can be used as a soil conditioner, a nitrification inhibitor, and a fertilizer, particularly in tropical zones to temperate zones.

Since zeanone is secreted from maize roots, it is possible to inhibit soil nitrification by cultivating maize. In addition, zeanone has low volatility and is not easily affected by the environmental temperature.

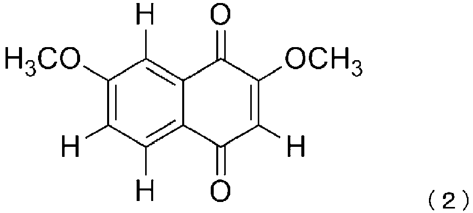

The 1,4-naphthoquinone derivatives are a group of compounds known to be contained in plants and microorganisms in the related art, and they are known to have various physiological activities. However, there has been no report that zeanone having a methoxy group at the 2-position and the 7-position has been isolated as a compound of natural origin. In addition, in the related art, there has been no report that zeanone has a nitrification action.

2,7-Dimethoxy-1,4-naphthoquinone (zeanone) or the 1,4-naphthoquinone derivative shown in Table 1 is added to inorganic materials such as lime or fertile soil such as andosol, thereby being capable of serving as a soil conditioner.

The amount thereof added may be appropriately selected and determined as necessary. The amount of zeanone added is, for example, in a range of 15 to 50 µg per 1 g of soil.

As will be described later in Examples, the nitrification inhibitory activity of the 1,4-naphthoquinone derivative shown in Table 1 is equal to or higher than that of zeanone. The amount of these derivatives added may be determined with reference to the nitrification inhibitory activity of each derivative. The amount of the derivative added per 1 g of soil may be, for example, 0.5 µg or more, 2 µg or more, 5 µg or more, 10 µg or more, and 20 µg or more, and it may be 50 µg or less, 20 µg or less, 10 µg or less, 5 µg or less, and 2 µg or less. Any combination of the upper limit value and the lower limit value of the amount of the derivative added can be used.

Since the soil conditioner containing 2,7-dimethoxy-1,4-naphthoquinone (zeanone) or the 1,4-naphthoquinone derivative shown in Table 1 has a nitrification inhibitory action, it can inhibit the nitrification of the nitrogen component and prevent the deterioration of the soil environment. The effect obtained using the soil conditioner according to the present embodiment may include an effect other than the nitrification action as long as it is an effect of promoting the growth of the plant body.

### [Fertilizer]

In one embodiment, the present invention provides a fertilizer containing, as a soil conditioner, the compound represented by General Formula (1) or a solvate of the compound or a salt thereof.

Since the fertilizer contains the above-described soil conditioner, it has a nitrification inhibitory action. This makes it possible to inhibit the nitrification of the nitrogen component and prevent the deterioration of the soil environment.

Examples of the fertilizer containing the above-described soil conditioner include an inorganic fertilizer and an organic fertilizer, and a mixed fertilizer thereof may be also used. As the inorganic fertilizer, a nitrogenous fertilizer such as urea, ammonium sulfate, or ammonium chloride, a phosphate fertilizer such as calcium superphosphate, and a potassium fertilizer such as potassium sulfate or potassium chloride can be used. Further, as the organic fertilizer, bone meal, compost, and the like can be used.

### [Production method]

In one embodiment, the present invention provides a production method for an active ingredient of the soil conditioner, which includes soaking a root surface part of maize in an organic solvent to obtain a root exudate and purifying an active ingredient of the soil conditioner contained in the root exudate by chromatography.

As will be described later in Examples, the inventors of the present invention separated zeanone having a nitrification inhibitory action, from the maize roots. Since zeanone is a hydrophobic compound, zeanone can be extracted into an organic solvent by immersing maize roots in an organic solvent.

Examples of the organic solvent that is used for extraction include alcohol, acetonitrile, diethyl ether, dichloromethane, chloroform, and ethyl acetate. From the relationship between the purity and the recovery rate of the extracted compound, a case of using acetic acid-containing dichloromethane has the best efficiency.

Examples of the method of purifying the soil conditioner from the organic solvent exuded from the maize roots include, but are not limited to, partition chromatography, normal phase chromatography, and reverse phase chromatography, where a known chromatography may be used as long as zeanone, which is a hydrophobic substance, can be purified. The obtained organic solvent extraction liquid can be fractionated by partition adsorption column chromatography to obtain 2,7-dimethoxy-1,4-naphthoquinone (zeanone).

2,7-Dimethoxy-1,4-naphthoquinone (zeanone) and the 1,4-naphthoquinone derivative shown in Table 1 exhibit an excellent nitrification inhibitory action, and can be added to a soil conditioner, a fertilizer, and the like, as a nitrification inhibitor. In addition, 2,7-dimethoxy-1,4-naphthoquinone (zeanone) used as a nitrification inhibitor can be produced from maize. As a result, it is possible to produce a soil conditioner containing a nitrification inhibitor at a low cost, and it is expected that the activity of the nitrification inhibitor will be exhibited where nitrification occurs in the soil.

### [Nitrification inhibition method]

In one embodiment, the present invention provides a nitrification inhibition method for soil, including cultivating a plant that generates an active ingredient of the soil conditioner.

In soil where maize is cultivated, soil nitrification can be naturally inhibited by zeanone being exuded into the soil. This makes it possible to prevent the deterioration of the soil environment.

As will be described later in Examples, the content of zeanone varies greatly depending on the maize strain. In a case of cultivating maize containing a large amount of zeanone, it is possible to carry out agricultural production while inhibiting the nitrification of the nitrogen component of the soil and preventing the deterioration of the soil environment.

Since maize is cultivated over large areas, it is expected that nitrification can be inhibited in vast areas of cultivated land by cultivating maize containing a large amount of the active ingredient of the soil conditioner.

In addition, the plant that generates the active ingredient of the soil conditioner is not limited to maize, and any plant may be used as long as it contains a large amount of the active ingredient of the soil conditioner.

### [Examples]

The present invention will be described with reference to Examples; however, the present invention is not limited to Examples below.

### [Experimental Example 1]

### (Separation and purification of zeanone)

From the extraction liquid obtained from the surface of the roots of cultivated maize, a compound having a nitrification inhibitory activity was separated and purified, and the structure thereof was determined.

The maize plant bodies were grown in a greenhouse for 20 days, and the roots of 200 maize plant bodies were immersed in 200 mL of dichloromethane containing 10% acetic acid to obtain a hydrophobic extraction liquid from the surface of the roots.

Next, this extraction liquid was concentrated with a rotary evaporator, the concentrate was dissolved in a small amount of methanol, and this was fractionated with a reverse phase solid-phase extraction cartridge (Sep-Pak C18 plus, manufactured by Waters Corporation). The activity was detected in the 50% methanol fraction. The activity was measured according to a method described later in Experimental Example 2.

This active fraction was further fractionated by high-performance liquid chromatography connected to a column (TSKgel Super ODS, manufactured by Tosoh Corporation), and finally, one active substance was purified.

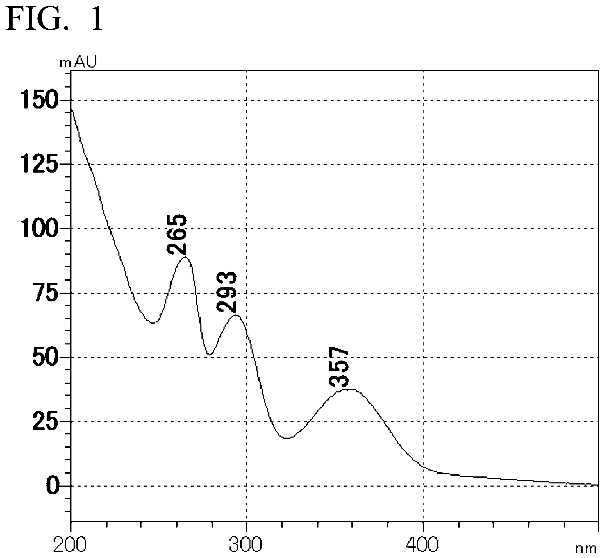

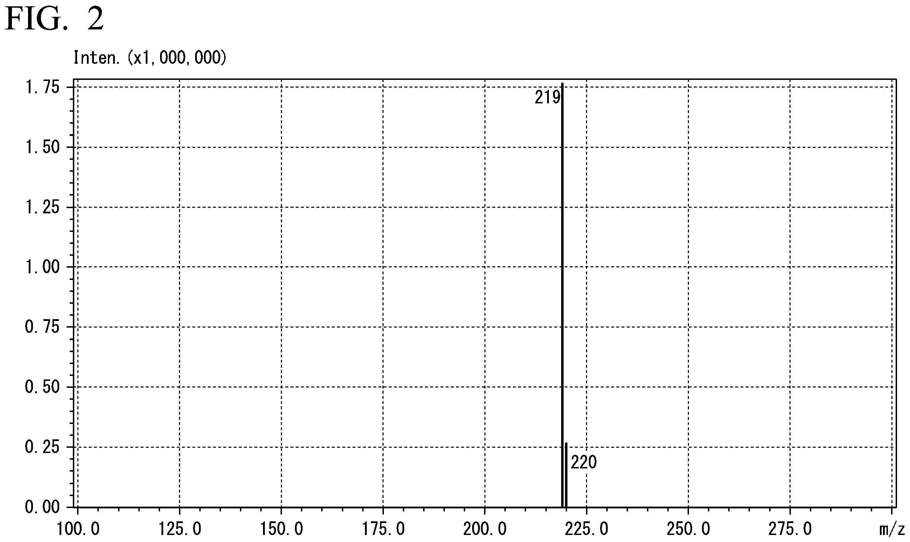

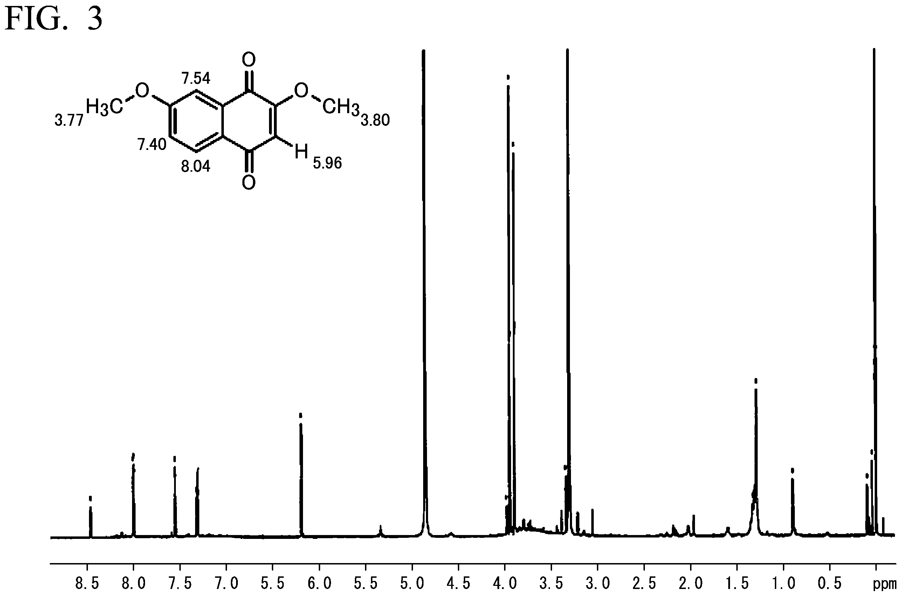

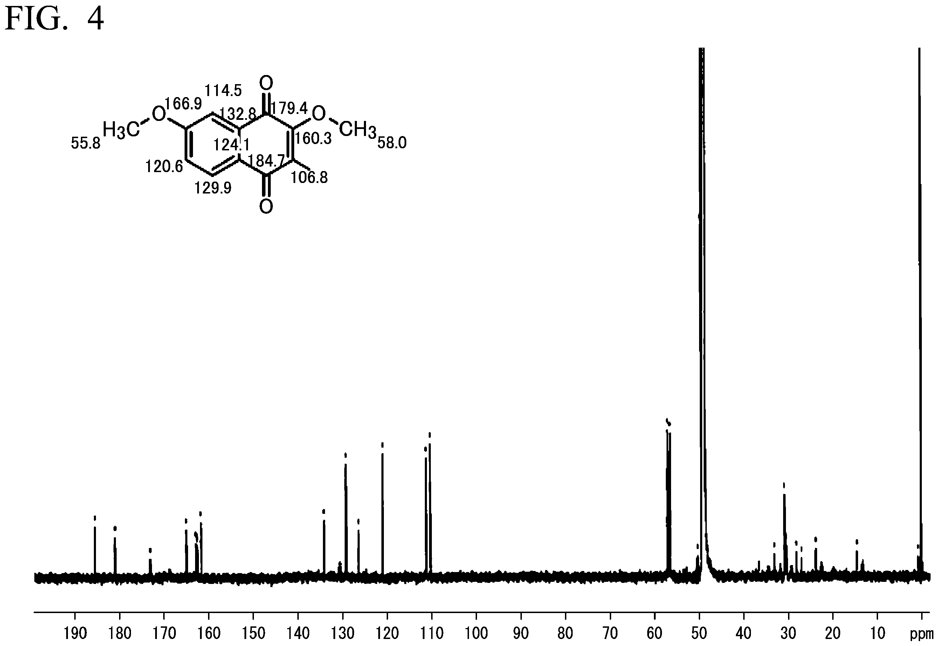

The obtained compound was subjected to an ultraviolet spectroscopic analysis, an electrospray ionization mass spectrometric analysis, a ¹H-NMR analysis, and a ¹³C-NMR analysis, and spectra therefrom were analyzed. These results are shown in FIG. 1 to FIG 4. FIG. 1 is an ultraviolet visible absorption spectrum, FIG. 2 is an electrospray ionization mass spectrum, FIG. 3 is a ¹H-NMR spectrum, and FIG. 4 is a ¹³C-NMR spectrum.

In addition, 2,7-dimethoxynaphthalene was oxidized with chromium acid to synthesize 2,7-dimethoxy-1,4-naphthoquinone (for example, R.D. Wilson, Naphthaquinones - I: The molecular structures of some halogeno-2:7-dihydroxynaphthalenes (1958) Tetrahedron, 3(3), 236-242).

The ultraviolet visible absorption spectrum, electrospray ionization mass spectrum, ¹H-NMR spectrum, and ¹³C-NMR spectrum of the active substance obtained from the extraction liquid of the maize root surface were consistent with the spectra of the chemically synthesized 2,7-dimethoxy-1,4-naphthoquinone. From these results, it was revealed that the active substance obtained from the extraction liquid of the maize root surface was 2,7-dimethoxy-1,4-naphthoquinone.

This is the first example of 2,7-dimethoxy-1,4-naphthoquinone in nature being isolated.

### [Experimental Example 2]

### (Nitrification inhibitory action of zeanone)

The nitrification inhibitory action of 2,7-dimethoxy-1,4-naphthoquinone was analyzed using a nitrifying bacterium in vitro (T. Iizumi, M. Mizumoto, K. Nakamura, A Bioluminescence assay using Nitrosomonas europaea for rapid and sensitive detection of nitrification inhibitors (1998) Appl. Environment. Microbiol., 64, 3656-3662).

A nitrifying bacterium (Nitrosomonas europaea IFO14298) into which a luciferase gene derived from a bacterium (luxAB) had been introduced was aerobically cultured at 30°C for 7 to 9 days in a P culture medium containing kanamycin (25 mg/1 L). The obtained nitrifying bacterium was washed and then suspended in a fresh P culture medium to prepare a nitrifying bacterial suspension. This nitrifying bacterial suspension was allowed to stand in a dark place for 30 minutes or more before the experiment.

Here, the composition of the P culture medium consisted of 2.5 g of (NH₄)₂SO₄, 0.7 g of KH₂PO₄, 13.5 g of Na₂HPO₄, 0.5 g of NaHCO₃, 100 mg of MgSO₄/7H₂O, 5 mg of CaCl₂/2H₂O, 1 mg of Fe-EDTA, and 1 L of water, and the pH thereof was 8.0.

After mixing in vitro an aqueous solution of the above-described nitrifying bacterial suspension, consisting of 0.25 mL of the above-described nitrifying bacterial suspension and 0.2 mL of water, with 0.01 mL of a sample solution of 2,7-dimethoxy-1,4-naphthoquinone at various concentrations, the amount of bioluminescence associated with the nitrification reaction during culturing at 15°C for 30 minutes was measured using a luminometer (manufactured by Turner Designs, Inc., model name: TD20/20), whereby the nitrification action was evaluated.

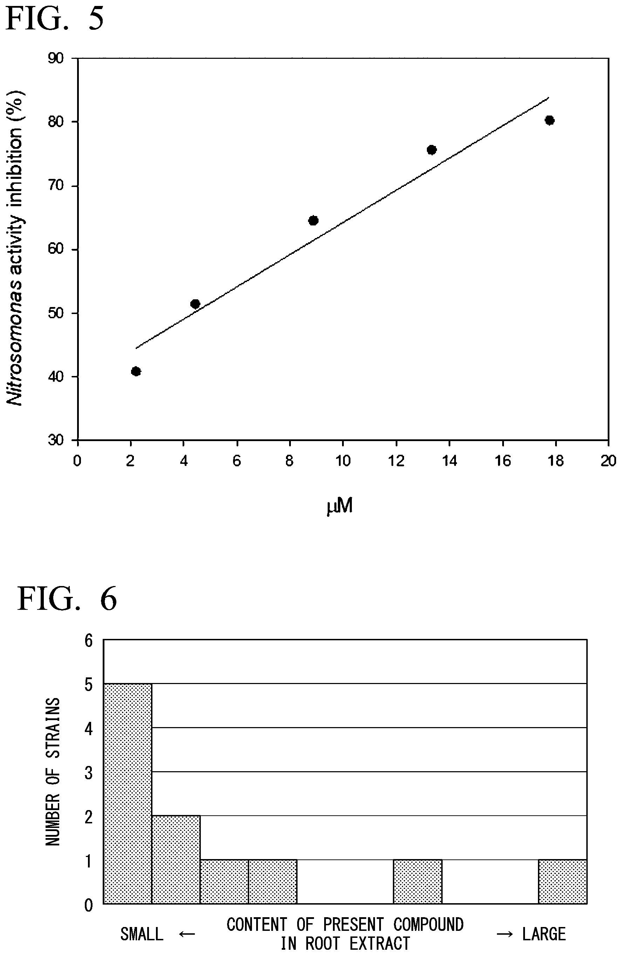

The amount of bioluminescence associated with the nitrification reaction decreases in a case where a substance having a nitrification inhibitory action is present in the sample solution. Therefore, the amount of luminescence in a case where each of the sample solutions of 2,7-dimethoxy-1,4-naphthoquinone at various concentrations was added to the aqueous solution of the nitrifying bacterial suspension was divided by the amount of luminescence in the case of only using the aqueous solution of the bacterial cell suspension without the sample solution being added, thereby obtaining a value as the nitrification inhibition rate. The results of measuring the nitrification inhibitory activity of the present compound at various concentrations are shown in FIG. 5. From this result, it was revealed that ED₅₀ was 4.2 µM and ED₈₀ was 16.1 µM.

In addition, the nitrification inhibitory activity of the 1,4-naphthoquinone derivative was measured in the same manner. The results are shown in Table 2. In Table 2, R¹ to R⁶ are each R¹ to R⁶ in Formula (1), respectively.

**[Table 2]**

| Substance name | R¹ | R² | R³ | R⁴ | R⁵ | R⁶ | Nitrification inhibitory activity ED₈₀ (µM) |
|---|---|---|---|---|---|---|---|
| 1,4-Naphthoquinone | H | H | H | H | H | H | 4.4 |
| 2-Methyl-1,4-naphthoquinone | CH₃ | H | H | H | H | H | 4.4 |
| 5-Hydroxy-1,4-naphthoquinone | H | H | OH | H | H | H | 1.1 |
| 5,8-Dihydroxy-1,4-naphthoquinone | H | H | OH | H | H | OH | 2.2 |
| 5-Hydroxy-2-methyl-1,4-naphthoquinone | CH₃ | H | OH | H | H | H | 0.5 |
| 2,3-Dichloro-1,4-naphthoquinone | Cl | Cl | H | H | H | H | 2.2 |
| 2-Methoxy-1,4-naphthoquinone | CH₃O | H | H | H | H | H | 8.9 |
| 2-Amino-3-chloro-1,4-naphthoquinone | NH₂ | Cl | H | H | H | H | 13.3 |
| 2-Hydroxy-1,4-naphthoquinone | OH | H | H | H | H | H | >100 |
| 2,3-Dichloro-5,8-dihydroxy-1,4-naphthoquinone | Cl | Cl | OH | H | H | OH | 0.9 |
| 6-Methyl-1,4-naphthoquinone | H | H | H | CH₃ | H | H | 2.2 |
| 2-Chloro-1,4-naphthoquinone | Cl | H | H | H | H | H | 4.0 |
| Zcanonc | CH₃O | H | H | H | CH₃O | H | 16.1 |

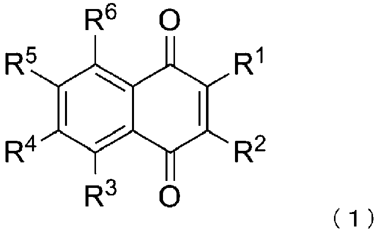

[In Formula (1), R¹ to R⁶ each independently represents a hydrocarbon group having 1 to 5 carbon atoms, which may have a substituent, an alkoxy group having 1 to 5 carbon atoms, which may have a substituent, an amino group, a hydroxy group, a halogen atom, or a hydrogen atom.]

From these results, it was revealed that 1,4-naphthoquinone, 2-methyl-1,4-naphthoquinone, 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2-methoxy-1,4-naphthoquinone, 2-amino-3-chloro-1,4-naphthoquinone, 2,3-dichloro-5,8-dihydroxy-1,4-naphthoquinone, 6-methyl-1,4-naphthoquinone, 2-chloro-1,4-naphthoquinone, and 2,7-dimethoxy-1,4-naphthoquinone (zeanone) have a nitrification inhibitory activity.

In addition, it was also revealed that 2-hydroxy-1,4-naphthoquinone does not have a nitrification inhibitory activity.

### [Experimental Example 3]

### (Zeanone content of maize)

Eleven strains of randomly selected maize (International Maize and Wheat Improvement Center) were cultivated, and the content of 2,7-dimethoxy-1,4-naphthoquinone contained in the hydrophobic extraction liquid obtained from the roots was analyzed.

Maize seeds of strain numbers 17528, 17529, 17531, 17540, 17542, 17544, 17546, 17548, 17549, 17550, and 17556 were ordered from the International Maize and Wheat Improvement Center. The seeds were grown in a greenhouse for 20 days, and the obtained roots of the maize plant body were immersed in 20 mL of dichloromethane containing 10% acetic acid to obtain a hydrophobic extraction liquid from the root surface.

The extraction liquid was filtered through a 0.45 µm filter, concentrated and dried to a solid, and dissolved in 1 mL of acetonitrile to prepare a sample. 10 µL of the sample was analyzed by a high-performance liquid chromatography-mass spectrometer connected to a column (TSKgel Super ODS, manufactured by Tosoh Corporation).

For each sample, the area of the peak of m/z = 219, which is the molecular ion peak of 2,7-dimethoxy-1,4-naphthoquinone, was calculated, and 2,7-dimethoxy-1,4-naphthoquinone content contained in each strain was estimated. The results are shown in Table 6.

FIG. 6 shows the content of 2,7-dimethoxy-1,4-naphthoquinone contained in the hydrophobic extraction liquid obtained from the roots of 11 strains of maize. In FIG. 6, the horizontal axis indicates the content of 2,7-dimethoxy-1,4-naphthoquinone (the present compound), and the vertical axis indicates the number of maize strains within each content range.

As a result, it was revealed that the content of 2,7-dimethoxy-1,4-naphthoquinone varies greatly depending on the maize strain.

### [Industrial Applicability]

According to the present invention, it is possible to inhibit soil nitrification with a compound capable of being used over wide areas from tropical zones to temperate zones and which can be easily obtained from a material of natural origin, or a derivative of the compound, and to provide a soil conditioner, a nitrification inhibitor, a fertilizer, and a nitrification inhibition method which uses these.

Further, according to the present invention, it is possible to provide a nitrification inhibitor having high utilization efficiency and low environmental load as compared with nitrogen fertilizers in the related art.

## Claims

1. A soil conditioner comprising, as an active ingredient, a compound represented by Formula (1) or a salt thereof, or a solvate of the compound or the salt thereof, 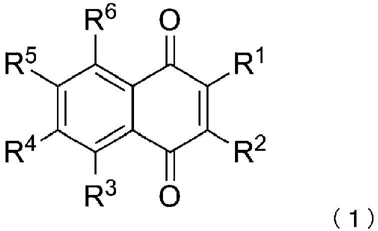 [in Formula (1), R¹ to R⁶ each independently represents a hydrocarbon group having 1 to 5 carbon atoms, which may have a substituent, an alkoxy group having 1 to 5 carbon atoms, which may have a substituent, an amino group, a hydroxy group, a halogen atom, or a hydrogen atom].

2. The soil conditioner according to Claim 1,
wherein the compound represented by Formula (1) is one or more selected from the group consisting of 1,4-naphthoquinone, 2-methyl-1,4-naphthoquinone, 5-hydroxy-1,4-naphthoquinone, 5,8-dihydroxy-1,4-naphthoquinone, 5-hydroxy-2-methyl-1,4-naphthoquinone, 2,3-dichloro-1,4-naphthoquinone, 2-methoxy-1,4-naphthoquinone, 2-amino-3-chloro-1,4-naphthoquinone, 2,3-dichloro-5,8-dihydroxy-1,4-naphthoquinone, 6-methyl-1,4-naphthoquinone, 2-chloro-1,4-naphthoquinone, and 2,7-dimethoxy-1,4-naphthoquinone.

3. The soil conditioner according to Claim 1 or 2,
wherein the soil conditioner has an effect of inhibiting nitrification.

4. A fertilizer comprising the soil conditioner according to any one of Claims 1 to 3.

5. A production method for an active ingredient of the soil conditioner according to any one of Claims 1 to 3, the production method comprising:
soaking a root surface part of a maize in an organic solvent to obtain a root exudate; and
purifying the root exudate by chromatography.

6. A nitrification inhibition method for soil, comprising cultivating a plant that generates an active ingredient of the soil conditioner according to any one of Claims 1 to 3.
